# EUROPÄISCHE PATENTANMELDUNG

(11) **EP 3 539 420 A1**
(43) Veröffentlichungstag der Anmeldung: **18.09.2019**
(21) Anmeldenummer: 18162381.0
(22) Anmeldetag: 16.03.2018
(51) Int. Cl.: A47B 81/00, B01L 1/02, C12M 1/00, E05C 19/16

(54) **LABORSCHRANKVORRICHTUNG ZUM LAGERN VON LABORPROBEN MIT MAGNETVERSCHLUSS**

(71) Anmelder: Eppendorf AG, 22339 Hamburg (DE)
(72) Erfinder: Schafrinski, Arne, 22339 Hamburg (DE)
(74) Vertreter: Kirchner, Christian

(57) **Zusammenfassung**

Die Erfindung betrifft eine Laborschrankvorrichtung zum Lagern von Laborproben mit Magnetverschluss für die Türe. Sie betrifft insbesondere einen Temperierschrank für das Temperieren von Laborproben, insbesondere einen Inkubator für das Wachstum von Zellkulturen. Der Magnetverschluss arbeitet mit einem vorteilhaft gestalteten Betätigungselement.

## Beschreibung

Die Erfindung betrifft eine Laborschrankvorrichtung zum Lagern von Laborproben mit Magnetverschluss für die Türe. Sie betrifft insbesondere einen Temperierschrank für das Temperieren von Laborproben, insbesondere einen Inkubator für das Wachstum von Zellkulturen.

Mit solchen Inkubatoren werden in biologischen und medizinischen Laboratorien Zellen in Zellkultur unter kontrollierten Umgebungsbedingungen gehalten, und so das Wachstum lebender Zellen *in vitro* ermöglicht. Dazu werden die Temperatur und die Gaszusammensetzung bzw. die Luftfeuchtigkeit der Atmosphäre im Inneren einer von der Umgebung isolierten Inkubatorkammer durch die apparativen Einrichtungen des Inkubators auf den gewünschten Werten gehalten. Eukaryotische Zellen benötigen CO₂-Inkubatoren. Die Atmosphäre wird durch Luft mit einem bestimmten CO₂- und O₂-Gehalt und einer bestimmten Luftfeuchtigkeit gebildet, eine geeignete Temperatur ist oftmals 37 °C. Solche Temperierschränke weisen eine Kammer zur Lagerung der Laborproben auf, mit einer Kammeröffnung, durch die der Benutzer die Proben im Kammerinneren lagert und wieder entnimmt.

Die Kammertüre soll das Kammerinnere zuverlässig schließen. Zu diesem Zweck sind im Stand der Technik verschiedene technische Lösungen bekannt. Mechanisch wirkende Verschlüsse weisen beispielsweise einen Riegelverschluss, einen Spann- oder Rastverschluss auf, die zum Öffnen entriegelt werden müssen. Solche mechanischen Lösungen sind aufwändig und erzeugen mechanische Vibrationen am Gehäuse. Eine einhändige Bedienung ist bei bekannten Riegelverschlüssen oftmals nur schwer möglich, wäre aber wünschenswert. In der Praxis hält der Anwender in einer Hand ein Probengefäß, das er dem Kammerinneren entnommen hat oder das er in das Kammerinnere einsetzen will. Somit hat der Anwender typischer Weise nur eine Hand zur Bedienung eines Verschlussmechanismus der Kammertüre frei. Da ein Verschütten oder zu starke Bewegung der meist flüssigen Laborprobe im Probengefäß vermieden werden muss, sollte die Bedienung des Verschlusses intuitiv und sicher erfolgen können und der Anwender sollte sein Hauptaugenmerk auf die sichere Handhabung der Probe richten können.

Um die gelagerten Laborproben zu schützen, ist es bei Laborschrankvorrichtungen bzw. bei Temperierschränken insbesondere bedeutsam, die Zeit zu minimieren, während der der Innenraum des gegenüber der Umgebung exponiert ist. Der vorliegenden Erfindung liegt die Beobachtung zugrunde, dass die Öffnungszeitintervalle durch die Gestaltung des Schließmechanismus beeinflussbar sind, mit dem die Kammertüre in der Schließposition gehalten und verschlossen wird. Es wurde deshalb eine Laborschrankvorrichtung mit einer Verschlusseinrichtung entwickelt, die vom Benutzer effizient zu bedienen ist, um die Öffnungszeit zu minimieren.

Der vorliegenden Erfindung liegt deshalb die Aufgabe zugrunde, eine Laborschrankvorrichtung anzugeben, deren Kammertürverschluss effizient zu bedienen ist.

Die Erfindung löst diese Aufgabe durch die Laborschrankvorrichtung gemäß Anspruch 1. Bevorzugte Ausgestaltungen sind insbesondere Gegenstände der Unteransprüche.

Durch die Ausgestaltung der Verschlusseinrichtung der Laborschrankvorrichtung, insbesondere durch die Gestaltung des Betätigungselements mit einem Grifflaschenabschnitt und der Möglichkeit, die magnetische Haltekraft sowohl durch Schwenken des Betätigungselements als auch durch Ziehen am Betätigungselement zu überwinden, wird eine effizient bedienbare Laborschrankvorrichtung realisiert. Da der Nutzer immer die Wahl hat, die Verschlusseinrichtung entweder durch Schwenken oder durch Ziehen zu öffnen, ist ein mit der erfindungsgenmäßen Verschlusseinrichtung ausgestatteter Inkubator sowohl von Rechtshändlern als auch von Linkshändlern immer effizient bedienbar. Durch die Verwendung der magnetisch miteinander wirkenden Halteelemente entfällt die Anforderung herkömmlicher, durch mechanischen Formschluss wirkender Riegelverschlüsse, das Betätigungselement vor dem Öffnen der Kammertüre erst schwenken zu müssen bzw. das Betätigungselement vor dem Schließen der Kammertüre in einer geschwenkten Position halten zu müssen. Der Grifflaschenabschnitt erlaubt eine intuitive Bedienung des Betätigungselements. Nahezu jede vom Benutzer intuitiv getätigte Bewegung des Grifflaschenabschnitts führt zum gewünschten Öffnen bzw. Schließen des magnetischen Verschlusses. Während der Betriebszeit des Inkubators wird somit die Zeit reduziert, die die unterschiedlichen Benutzer benötigen, um die Bedienung des Kammertürverschlusses zu erlernen und zu vollziehen. Insbesondere ist eine einhändige Bedienbarkeit der Verschlusseinrichtung erleichtert, da die Bedienbarkeit unabhängig ist von den subjektiven Präferenzen des Benutzers, der Bedienung mit der rechten oder linken Hand oder auch mit einem anderen Körperteil oder Hilfsmittel, und unabhängig davon ob die Verschlusseinrichtung an der linken Seite oder rechten Seite der Kammertüre montiert ist. Die Zeit, während der der Innenraum der Kammer gegenüber der Umgebung exponiert ist, wird durch die Verschlusseinrichtung minimiert. Die Verschlusseinrichtung kann optimal in die Laborschrankvorrichtung integriert werden, da der Platzbedarf der Verschlusseinrichtung an der Laborschrankvorrichtung aufgrund der kompakten Raumanforderungen des Grifflaschenabschnitts gering ist.

Die Laborschrankvorrichtung zum Lagern von Laborproben ist insbesondere ein Temperierschrank zum Temperieren von Laborproben. Solche Geräte werden elektrisch betrieben und weisen einen Spannungsanschluss auf. Die Halteeinrichtung arbeitet vorzugsweise mit einem oder mehreren Permanentmagneten, so dass die Halteeinrichtung unabhängig von der Spannungsversorgung ist. Der Verschluss der Gehäusetüre ist deshalb auch bei einem Ausfall der Spannungsversorgung gewährleistet. Anstelle der magnetisch miteinander wirkenden Halteelemente kann auch eine Verbindungseinrichtung verwendet werden, die eine Klemmverbindung, insbesondere eine Rastverbindung bereitstellt. Eine solche Klemmverbindung kann insbesondere so eingerichtet sein, dass sie sowohl durch Schwenken des Betätigungselements als auch durch Ziehen am Betätigungselement überwindbar ist. Der Gegenstand der Erfindung bezieht sich deshalb auch auf eine Laborschrankvorrichtung, deren Verschlusseinrichtung alternativ und/oder zusätzlich zu den magnetisch miteinander wirkenden Halteelementen (erstes und zweites Halteelement) mindestens zwei über eine Klemmverbindung verbindbare Halteelemente aufweist.

Der Temperierschrank temperiert die Laborproben, das heißt, er hält das Kammerinnere und damit die dort lagernden Laborproben im Rahmen von Toleranzen durch eine Temperaturregelung auf einer insbesondere vom Benutzer einstellbaren Solltemperatur.

Diese kann über der Raumtemperatur (Umgebungstemperatur) liegen, wie dies bei einem Wärmeschrank oder Inkubator der Fall ist, oder kann unter der Raumtemperatur liegen, wie dies bei einem Kühlschrank oder Gefrierschrank der Fall ist. Bei einer als Klimaschrank ausgebildeten Laborschrankvorrichtung wird vorzugsweise auch ein im Inneren der Kammer vorherrschender Klimaparameter im Rahmen von Toleranzen geregelt. Dieser Klimaparameter kann die Luftfeuchtigkeit sein, und/oder eine Gaskonzentration, z.B. eine CO₂, N2 und/oder O₂-Konzentration. Ein solcher Klimaschrank ist beispielsweise ein Inkubator für aus lebenden Zellkulturen bestehende Laborproben.

Das Betätigungselement ist vorzugsweise ein plattenförmiges, vorzugsweise im Wesentlichen flaches, Bauteil. Das Betätigungselement ist insbesondere nicht größer als ein Quader mit den Abmessungen (Länge x Breite x Höhe) von vorzugsweise 20 cm x 10 cm x 4 cm, vorzugsweise 15 cm x 6 cm x 3 cm. Das Betätigungselement weist vorzugsweise mindestens eine Aussparung und/oder einen Hohlraum auf. Eine solche Aussparung bzw. ein solcher Hohlraum lässt sich vorteilhaft zur Aufnahme weiterer funktioneller Bestandteile nutzen und reduziert das Gewicht und die Herstellungskosten. Das Betätigungselement weist vorzugsweise einen langgestreckten Basiskörper, insbesondere Plattenkörper auf, der als erstes Ende den Grifflaschenabschnitt und ein zweites Ende aufweist. Zwischen den Enden des Basiskörpers ist vorzugsweise ein Loch oder eine Aussparung vorgesehen, durch die senkrecht zur Schwenkebene bzw. Plattenebene des Basiskörpers eine Drehachse geführt werden kann. Ein Drehachsenelement kann ein Metallstiftelement sein, das an der Kammertüre befestigt ist. Mittels des Drehachsenelements ist das Betätigungselement vorzugsweise schwenkbar an der Kammertüre befestigt.

Das an der Kammertüre befestigte Betätigungselement weist eine Schwenkebene auf, zur der das Betätigungselement stets parallel verläuft, wenn es zwischen der ersten und zweiten Schwenkposition geschwenkt wird. Das Betätigungselement weist eine Unterseite auf, die der Kammertüre zugewandt ist, und eine Oberseite, die der Kammertüre abgewandt ist und die insbesondere dem Benutzer zugewandt ist.

Der Grifflaschenabschnitt ist vorzugsweise integral mit dem Betätigungselement ausgebildet, so dass das Betätigungselement mechanisch belastbar ist und über die gesamte Lebensdauer der Laborschrankvorrichtung zuverlässig verwendbar ist. Der Grifflaschenabschnitt kann aber auch ein separates Teil sein, das mit dem Betätigungselement verbunden ist. Der Grifflaschenabschnitt kann insbesondere einen verformbaren bzw. weichen Teilabschnitt aufweisen. Der Teilabschnitt kann insbesondere aus einem Elastomer bestehen, z.B. Silikon, und kann als Abdeckung oder Hülle des Grifflaschenabschnitts oder des gesamten Betätigungselements ausgebildet sein. Durch ein Elastomer kann das Greifen des Grifflaschenabschnitts vereinfacht werden bzw. angenehmer sein.

Der Grifflaschenabschnitt weist vorzugsweise eine am ersten Ende des Betätigungselements ausgebildete Kante auf, die eine um die Drehachse verlaufende Biegung aufweist. Durch diese Ausgestaltung assoziiert ein Benutzer die Bedienbarkeit durch eine Schwenkbewegung, so dass die Bedienung des Betätigungselements intuitiv erfolgen kann. Die gebogene Kante kann insbesondere einem Kreisumfang folgen, der insbesondere konzentrisch zur Drehachse verlaufen kann.

Der Grifflaschenabschnitt weist vorzugsweise an seiner in Schließposition zur Frontseite des Kammergehäuses weisenden Unterseite das erste Halteelement auf. Das erste Halteelement kann auch bis zur Oberseite des Betätigungselements münden oder aus der Oberseite hervorstehen. Das erste Halteelement kann auch das erste Ende des Betätigungselements bilden.

Der Grifflaschenabschnitt weist vorzugsweise mindestens einen Laschenabschnitt auf, der in der zweiten Schwenkposition in der Schwenkebene des Betätigungselements über die Kante der Kammertüre hinausragt und zudem über die Ausdehnung des ersten Halteelements in dieser Schwenkebene hinausragt. Durch das Hinausragen des Laschenabschnitts in der Schwenkebene wird ein platzsparender Griff realisierbar, der eine zuverlässige und komfortable Bedienung des Betätigungselements und der gesamten Kammertüre ermöglicht.

Der Grifflaschenabschnitt kann insbesondere mindestens einen, insbesondere zwei, jeweils als Laschenabschnitte ausgebildete Flügelabschnitte aufweisen, die insbesondere integral mit dem Grifflaschenabschnitt bzw. dem Betätigungselement geformt sind. Die Flügelabschnitte springen gegenüber dem Plattenkörper des Betätigungselements parallel zur Schwenkebene des Plattenkörpers bzw. zur Hauptebene der Kammertüre seitlich ab, so dass ein Finger des Benutzers in der Schwenkposition P2 jeweils hinter dem ersten und/oder zweiten Flügelabschnitt eingreifen kann, um die Kammertüre durch Ziehen zu Öffnen. Der Grifflaschenabschnitt und die Flügelabschnitte springen insbesondere gegenüber dem ersten Halteelement parallel zur Schwenkebene des Plattenkörpers bzw. zur Hauptebene der Kammertüre seitlich ab, so dass das Hintergreifen des Grifflaschenabschnitts erleichtert wird. Diese besondere Ausgestaltung ist ergonomisch vorteilhaft, insbesondere ist so ein leicht zugänglicher Raum für die eingreifenden Finger geschaffen.

Das erste Halteelement kann ein Plattenelement sein, das in eine Aussparung auf der Unterseite des Grifflaschenabschnitts eingesetzt und eingeschraubt und/oder eingeklebt ist. Das Halteelement kann auch gefedert gelagert am Betätigungselement angeordnet sein, um eine Ausweichbewegung des Halteelements in Richtung senkrecht zur Schwenkebene des Plattenkörpers bzw. zur Hauptebene der Kammertüre zu ermöglichen.

In der Schließposition der Kammertüre und in der Schwenkposition P2 des Betätigungselements liegen das erste und zweite Halteelement aneinander an und haften aufgrund der magnetischen Anziehungskraft - und/oder aufgrund einer Klemmkraft - aneinander. Durch Drehen des Betätigungselements aus der Position P2 in die Position P1 lässt sich diese Anziehungskraft leichter und komfortabler überwinden als durch Ziehen in Richtung A. In der geöffneten Position der Kammertüre, wenn das Betätigungselement in der Position P2 angeordnet ist, führt insbesondere das Schließen der Kammertüre automatisch zum Schließen des magnetischen Verschlusses, indem im Einflussbereich der Magnetkraft die magnetische Haftkraft das erste und zweite Halteelement direkt aufeinander zu bewegt. Die Verschlusseinrichtung ist vorzugsweise so eingerichtet, dass das Anschlagen der Halteelemente -insbesondere als "Klick"-akustisches wahrnehmbar ist, so dass der Benutzer ein akustisches Feedback über das erfolgreiche Schließen der Kammertüre erhält.

Die Verschlusseinrichtung weist vorzugsweise eine Positionsrasteinrichtung auf, mittels der das Betätigungselement zumindest in der zweiten Schwenkposition einrastet und/oder zumindest auch in einer ersten Schwenkposition einrastet. In der zweiten Schwenkposition ist das Betätigungselement vorzugsweise horizontal angeordnet, das heißt seine Längsachse L, erstreckt sich horizontal. In der ersten Schwenkposition ist das Betätigungselement vorzugsweise vertikal angeordnet, das heißt seine Längsachse L erstreckt sich vertikal.

Die Positionsrasteinrichtung weist mindestens ein Rastelement auf, vorzugsweise mehrere Rastelemente, vorzugsweise genau ein Rastelement, vorzugsweise genau zwei Rastelemente und besonders bevorzugt genau drei Rastelemente auf. Das oder die Rastelemente kann / können auf einem Trägerelement angeordnet sein, dass insbesondere als Rastelementenplatte ausgebildet sein kann, auf der mindestens ein Rastelement angeordnet ist. Das mindestens eine Rastelement wirkt mit mindestens einem Rastabschnitt zusammen, um eine Rastposition bzw. eine verrastete Schwenkposition zu realisieren. Der Rastabschnitt kann Bestandteil eines Höhenprofils sein, das in einem Höhenprofilabschnitt ausgebildet sein kann. Das mindestens eine Rastelement, insbesondere das Trägerelement, kann an der Kammertüre befestigt sein und der mindestens eine Rastabschnitt, insbesondere der Höhenprofilabschnitt, kann am Betätigungselement befestigt sein, insbesondere integral mit dem Betätigungselement ausgebildet sein. Auch die umgekehrte Konstellation ist möglich: das mindestens eine Rastelement kann am Betätigungselement befestigt sein und der mindestens eine Rastabschnitt kann an der Kammertüre befestigt sein.

Ein Rastelement kann einen in Richtung senkrecht zur Schwenkebene nach oben weisende Halterung für eine beweglich gelagertes Gleitelement, insbesondere eine Kugel, aufweisen. In der Halterung kann das Gleitelement gefedert gelagert angeordnet sein. Das Gleitelement ist dazu angepasst, in eine Rastvertiefung eines Rastabschnitts einzugreifen, der insbesondere in einem Höhenprofilabschnitt ausgebildet ist. Der Höhenprofilabschnitt ist vorzugsweise in einen Hohlraum bzw. in eine Aussparung der Unterseite des Betätigungselements integriert. Das Höhenprofil des Höhenprofilabschnitts ist vorzugsweise entlang einer Kreisringbahn verlaufend vorgesehen, die konzentrisch mit der Drehachse des Betätigungselements angeordnet ist. Eine erste Rastvertiefung kann vorgesehen sein, damit das Betätigungselement in der ersten Schwenkposition einrastet, eine zweite Rastvertiefung kann dazu vorgesehen sein, damit das Betätigungselement in der zweiten Schwenkposition einrastet. Das Höhenprofil ist zudem vorzugsweise so ausgebildet, dass die Halterung des Gleitelements an vorbestimmten erhöhten Positionen des Höhenprofils anschlägt, so dass das Betätigungselement, wenn dieses bestimmungsgemäß an der Kammertüre montiert ist, nur zwischen den diesen erhöhten Positionen entsprechenden Schwenkpositionen, und nicht darüber hinaus, schwenkbar ist. Die erste und zweite Schwenkposition stimmen vorzugsweise jeweils mit den diesen erhöhten Positionen entsprechenden Schwenkpositionen überein. Das korrekte Schließen der Verschlusseinrichtung wird für den Benutzer insbesondere auch durch ein Verrasten der Positionsrasteinrichtung wahrnehmbar.

Vorzugsweise ist die Positionsrasteinrichtung, insbesondere deren Höhenprofil, dazu eingerichtet, wahlweise so montiert zu werden, dass ein am rechten Rand der Kammertüre befestigtes Betätigungselement aus der zweiten Schwenkposition nur entgegen dem Uhrzeigersinn bis zur ersten Schwenkposition schwenkbar ist, oder dass ein am linken Rand der Kammertüre befestigtes Betätigungselement aus der zweiten Schwenkposition nur im Uhrzeigersinn bis zur ersten Schwenkposition schwenkbar ist.

Ein weiteres bevorzugtes Merkmal ist, dass das Betätigungselement und insbesondere dessen Positionsrasteinrichtung, also insbesondere das Höhenprofil sowie die dazu komplementäre Rastelementenplatte, so eingerichtet sind, dass das Betätigungselement und insbesondere seine Rastelementenplatte wahlweise am linken oder rechten Rand einer Türe montiert werden kann. Dazu werden insbesondere äquidistant entlang des Höhenprofils verteilte Rastvertiefungen und/oder Erhöhungen vorgesehen. Aufgrund dieser Eigenschaft ist das Betätigungselement bzw. die so gestaltete erfindungsgemäße Laborschrankeinrichtung effizient und flexibel konstruierbar bzw. nachträglich zu ändern, da ein und dasselbe Betätigungselement in Situationen verwendbar ist, in denen entweder ein linksseitiges Türscharnier oder ein rechtsseitiges Türscharnier benötigt wird.

Die Laborschrankvorrichtung kann ein Gehäuse aufweisen, vorzugsweise ein äußeres Gehäuse, dessen Gehäusewände mit der Umgebung in Kontakt stehen. Das Kammergehäuse liegt dann vorzugsweise innerhalb des Außengehäuses. In diesem Fall kann ein Inkubator mindestens ein als inneres Gehäuse dienendes Kammergehäuse aufweisen, das durch mindestens eine Kammertüre verschließbar ist. Zusätzlich zur Kammertüre kann eine äußere Gehäusetüre vorgesehen sein, die in der Verschlussposition an die Umgebung grenzt, wobei nur die äußere Gehäusetüre in der Verschlussposition an die Umgebung grenzt und die Kammertüre in der Verschlussposition der äußeren Gehäusetüre in einem Hohlraum zwischen Kammergehäuse, äußerem Gehäuse und äußerer Gehäusetüre liegt. Die Verschlussposition wird auch als Schließposition bezeichnet.

Die Kammertüre weist insbesondere eine Scharniereinrichtung auf, welche die Kammertüre schwenkbar mit dem Kammergehäuse verbindet. Eine solche Schwenktüre wird durch eine Rotation zwischen einer geöffneten Position und der Verschlussposition bewegt. Die Scharniereinrichtung kann insbesondere an der - im bestimmungsgemäßen Gebrauch der Laborschrankvorrichtung - vertikal orientierten Außenkante eines quaderförmigen Kammergehäuses liegen, welche an die Kammeröffnung angrenzt. Die Bodenplatte eines quaderförmigen Kammergehäuses ist im bestimmungsgemäßen Gebrauch der Laborschrankvorrichtung horizontal angeordnet, die Seitenwände des Kammergehäuses sind insbesondere vertikal angeordnet, und die Deckplatte des Kammergehäuses ist insbesondere der Bodenplatte gegenüberliegend horizontal angeordnet. Die Verschlusseinrichtung ist vorzugsweise an einer Außenkante des Kammergehäuses angeordnet, an der nicht die Scharniereinrichtung angeordnet ist, vorzugsweise in Verschlussposition gegenüberliegend der Scharniereinrichtung. Eine Verschlusseinrichtung kann aber auch alternativ oder zusätzlich an einer anderen Außenkante des Kammergehäuses angeordnet sein, insbesondere einer horizontal verlaufenden Außenkante.

Die Kammertüre kann aber auch eine Schiebetüre sein, die durch eine translatorische Bewegung zwischen einer geöffneten Position und der Verschlussposition bewegt wird. Auch eine gemischt schwenkende/translatorische Bewegung der Kammertüre ist möglich.

Die Laborschrankvorrichtung kann mehr als eine Verschlusseinrichtung aufweisen, die jeweils, wie definiert, die in Verschlussposition der Kammertüre magnetisch miteinander wechselwirkende Halteelemente aufweist. Im Fall von mehreren Verschlusseinrichtungen sind diese vorzugsweise in Verschlussposition voneinander beabstandet angeordnet. Sie können insbesondere an derselben Außenkante des Kammergehäuses oder an unterschiedlichen Außenkanten des Kammergehäuses angeordnet sein. Durch die Bereitstellung mehrerer Verschlusseinrichtungen kann das gleichmäßige Schließen der Gehäusetüre und die Verteilung der zwischen Gehäusetüre und Kammergehäuse wirkenden Verschlusskräfte optimiert werden.

Vorzugsweise sind die mehreren Verschlusseinrichtungen in identischer Weise aus mindestens einem ersten und einem zweiten Halteelement bestehend ausgeführt. Es kann aber auch vorgesehen sein, dass die mehreren Verschlusseinrichtungen in unterschiedlicher Weise aus mindestens einem ersten und einem zweiten Halteelement bestehend ausgeführt sind. Dadurch kann die Verschlusscharakteristik der Gehäusetüre, also die mittels der Verschlusseinrichtungen bewirkte Kraft zwischen Kammergehäuse und Kammertüre, aufgetragen gegen die Auslenkungsstrecke der Kammertüre aus der Verschlussposition, in gewünschter Weise beeinflusst werden.

In der Verschlussposition verschließt die äußere Gehäusetüre das Gehäuseinnere vorzugsweise gasdicht, was insbesondere durch mindestens eine Dichtungseinrichtung der Gehäusetüre bzw. des Rahmens der Gehäuseöffnung gelingt. In der Verschlussposition verschließt die Kammertüre das Kammerinnere vorzugsweise gasdicht, was insbesondere durch mindestens eine Dichtungseinrichtung der Kammertüre bzw. des Rahmens der Kammeröffnung gelingt. Die Erfindung betrifft aber grundsätzlich auch Laborschrankvorrichtungen mit einem Gehäuse und/oder einem Kammergehäuse, das das Innere gegenüber der Umgebung nicht vollständig abdichtet.

Die Verschlusseinrichtung weist vorzugsweise eine Gruppe von magnetisch wirkenden Halteelementen auf, die das erstes Halteelement beinhaltet, das am Kammergehäuse angeordnet ist, insbesondere als separates Bauteil dort befestigt oder dort integriert ist, und ein zweites Halteelement, das an der Kammertüre angeordnet ist, insbesondere als separates Bauteil dort befestigt oder dort integriert ist. Die Gruppe von magnetisch wirkenden Halteelementen weist vorzugsweise genau zwei Halteelemente auf.

Die magnetische Wirkung der Halteelemente beruht darauf, dass mindestens ein Halteelement als Magnetelement ausgebildet ist, indem es mindestens einen Magneten aufweist oder aus mindestens einem Magneten besteht. Dieser ist in besonders bevorzugter Ausgestaltung ein Permanentmagnet. Es kann sich aber auch um einen Elektromagneten handeln.

Vorzugsweise weist die Gruppe von magnetisch wirkenden Halteelementen ein Magnetelement auf und insbesondere ein zu diesem komplementäres magnetisches Element zur Ausbildung der magnetischen Anziehungskraft auf, insbesondere mittels Ferromagnetismus. Eine Halteeinrichtung kann mehrere Magnetelemente aufweisen, die die gewünschte Magnetkraft erzeugen, insbesondere 2, 3, 4, 5, 6 Magnetelemente oder eine andere Anzahl. Durch eine solche Gestaltung kann die Verschlusscharakteristik präzise eingestellt und mit genau definierten Haltekräften wieder gelöst werden.

Ein Halteelement bzw. Magnetelement ist vorzugsweise ein separates Bauteil, das bei Montage der Laborschrankvorrichtung mit dem Kammergehäuse und/oder der Kammertüre befestigt wird. Ein Halteelement bzw. Magnetelement kann aber auch ein integraler Bestandteil des Kammergehäuses und/oder der Kammertüre sein.

Ein Magnetelement ist vorzugsweise ein Permanentmagnet oder weist einen solchen auf. Vorzugsweise weist der Magnet ein Abdeckelement auf, um ihn vor mechanischen Schäden oder vor Korrosion zu schützen.

Vorzugsweise ist der Permanentmagnet aus einer Samarium-Cobalt-Legierung hergestellt oder weist diese Materialien auf. Diese Materialien sind extrem temperaturbeständig und haben sich in feuchten bzw. in mit Chemikalien behafteten Laborumgebungen und Kammerinnenräumen, die insbesondere zu Sterilisationszwecken auf Temperaturen von bis zu 180° C erhitzt werden müssen, als widerstandsfähig gegen Korrosion erwiesen. Es ist aber auch möglich, andere Permanentmagneten zu verwenden.

Vorzugsweise weist die Laborschrankvorrichtung mindestens ein elastisches Element auf, das in der Verschlussposition zwischen Kammergehäuse und Kammertüre angeordnet und insbesondere in der Verschlussposition zwischen Kammergehäuse und Kammertüre durch die Haltekraft der Verschlusseinrichtung komprimiert ist. Das elastische Element dient insbesondere als Anschlag für die Kammertüre am Kammergehäuse, der den Kontakt der Kammertüre am Kammergehäuse mechanisch abfedert. Zudem dient das elastische Element als Widerlager für die Haltekraft der Verschlusseinrichtung, die das elastische Element in der Verschlussposition komprimiert.

Das elastische Element ist insbesondere eine Dichtung, die in der Verschlussposition das Kammerinnere bzw. das Gehäuseinnere abdichtend von der Umgebung abtrennt. Die Dichtung ist insbesondere an einer Außenwand des Gehäuses angeordnet, welche auch die Gehäuseöffnung aufweist, bzw. an der Außenwand des Kammergehäuses, welche auch die Kammeröffnung aufweist, also insbesondere die Frontseite. Die Dichtung läuft vorzugsweise durchgehend um die Gehäuseöffnung bzw. Kammeröffnung um. Alternativ oder zusätzlich kann eine solche Dichtung an der Innenseite der Gehäusetüre bzw. Kammertüre so angeordnet sein, dass sie in der Verschlussposition die Gehäuseöffnung bzw. die Kammeröffnung durchgehend umläuft. Die Dichtung weist vorzugsweise Silikon oder Fluorkautschuk auf bzw. besteht vorzugsweise aus Silikon oder Fluorkautschuk. Das Silikon oder der Fluorkautschuk (FKM) können geschäumt sein (Silikonschaum oder Fluorkautschukschaum) und/oder einen oder mehrere Hohlräume und/oder Aussparungen aufweisen. Durch solche Poren bzw. Hohlräume wird die gewünschte Elastizität bzw. die thermische Isolierfähigkeit der Dichtung erreicht.

Die Verschlussposition ist insbesondere dadurch gekennzeichnet, dass das Kammerinnere durch die Kammertüre verschlossen wird, insbesondere hermetisch verschlossen wird, und insbesondere mittels einer Dichtung hermetisch verschlossen wird. In der Verschlussposition wird das elastisch verformbare Element bzw. die die elastisch verformbare Dichtung durch die magnetische Haltekraft der Verschlusseinrichtung elastisch verformt.

Vorzugsweise weist wenigstens ein Halteelement einer Gruppe von Halteelementen einen Permanentmagneten auf und wenigstens ein weiteres Halteelement dieser Gruppe ein mit diesem Permanentmagneten magnetisch wechselwirkendes Material. Vorzugsweise weist das erste Halteelement einen Permanentmagneten auf und das zweite Halteelement ein mit diesem Permanentmagneten magnetisch wechselwirkendes Material. Vorzugsweise weist das zweite Halteelement einen Permanentmagneten auf und das erste Halteelement ein mit diesem Permanentmagneten magnetisch wechselwirkendes Material. Insbesondere dieses Material kann als Abschnitt der Kammertüre oder des Kammergehäuses ausgebildet sein.

Vorzugsweise weist wenigstens ein Halteelement einer Gruppe von Halteelementen einen ersten Permanentmagneten auf und wenigstens ein weiteres Halteelement dieser Gruppe ein mit diesem ersten Permanentmagneten magnetisch wechselwirkenden zweiten Permanentmagneten auf. Vorzugsweise weist das erste Halteelement einen ersten Permanentmagneten auf und das zweite Halteelement ein mit diesem ersten Permanentmagneten magnetisch wechselwirkenden zweiten Permanentmagneten auf. Der erste und der zweite Permanentmagnet sind in der Verschlussposition vorzugsweise mit gleichgerichteter Polung angeordnet, so dass zwischen dem ersten und dem zweiten Permanentmagneten eine anziehende Wirkung besteht.

Vorzugsweise ist der mindestens eine Permanentmagnet ein Bestandteil eines Halteelements der Gruppe von Halteelementen und insbesondere Bestandteil eines Magnetelements. Dieses weist vorzugsweise ein Abdeckelement oder eine Fassung auf, von der der mindestens eine Permanentmagnet teilweise oder vollständig eingefasst ist.

Weitere bevorzugte Ausgestaltungen der erfindungsgemäßen Laborschrankvorrichtung lassen sich der Beschreibung der Ausführungsbeispiele gemäß den Figuren entnehmen.

Es zeigen:
Fig. 1 zeigt eine erfindungsgemäße Laborschrankvorrichtung gemäß einem Ausführungsbeispiel in perspektivischer Ansicht.
Fig. 2 zeigt eine erfindungsgemäße Laborschrankvorrichtung gemäß einem Ausführungsbeispiel in Frontansicht.
Fig. 3a zeigt eine Verschlusseinrichtung, die bei einer erfindungsgemäßen Laborschrankvorrichtung gemäß Fig. 1 oder 2 zum Einsatz kommt, in einer perspektivischen Frontansicht in der zweiten Schwenkposition des Betätigungselements, geschlossene Schwenkposition (P2), Schließposition der Kammertür.
Fig. 3b zeigt die Verschlusseinrichtung der Fig. 3a, in einer perspektivischen Frontansicht in der ersten Schwenkposition des Betätigungselements und in geöffneten Position der Kammertüre.
Fig. 4a zeigt eine perspektivische Querschnittsansicht durch die Verschlusseinrichtung der Fig. 3b, wobei sich der Querschnitt senkrecht zur Schwenkebene des Betätigungselements und entlang dessen Längsachse (L) erstreckt.
Fig. 4b zeigt schematisch eine Aufsicht auf das Höhenprofil einer Rastpositionseinrichtung, das an der der Kammertüre zugewandten Unterseite des Betätigungselements in Fig. 3b ausgebildet ist.
Fig. 4c zeigt schematisch eine Aufsicht auf die Rastelementenplatte, die bei der Positionsrasteinrichtung in Fig. 4a verwendet wird.
Fig. 4d zeigt das Betätigungselement der Fig. 4a sowie dessen Höhenprofil und die fest an der Tür montierten Rastelemente in der ersten Schwenkposition bei Montage an einem rechten Rand der Tür, wie dies in Fig. 4a der Fall ist.
Fig. 4e zeigt das Betätigungselement der Fig. 4a sowie dessen Höhenprofil und die fest an der Tür montierten Rastelemente in der zweiten Schwenkposition bei Montage an einem rechten Rand der Tür.
Fig. 4f zeigt das Betätigungselement der Fig. 4a sowie dessen Höhenprofil und die gegenüber der Fig. 4d und 4e leicht verdreht an der Tür befestigten Rastelemente in der ersten Schwenkposition bei Montage an einem linken Rand der Tür.
Fig. 4g zeigt das Betätigungselement der Fig. 4a sowie dessen Höhenprofil und die gegenüber der Fig. 4d und 4e leicht verdreht an der Tür montierten Rastelemente in der zweiten Schwenkposition bei Montage an einem linken Rand der Tür.
Fig. 5 zeigt die Verschlusseinrichtung der Fig. 3a, in einer perspektivischen Seitenansicht und in der zweiten Schwenkposition des Betätigungselements und in einer geschlossenen Position der Kammertüre.

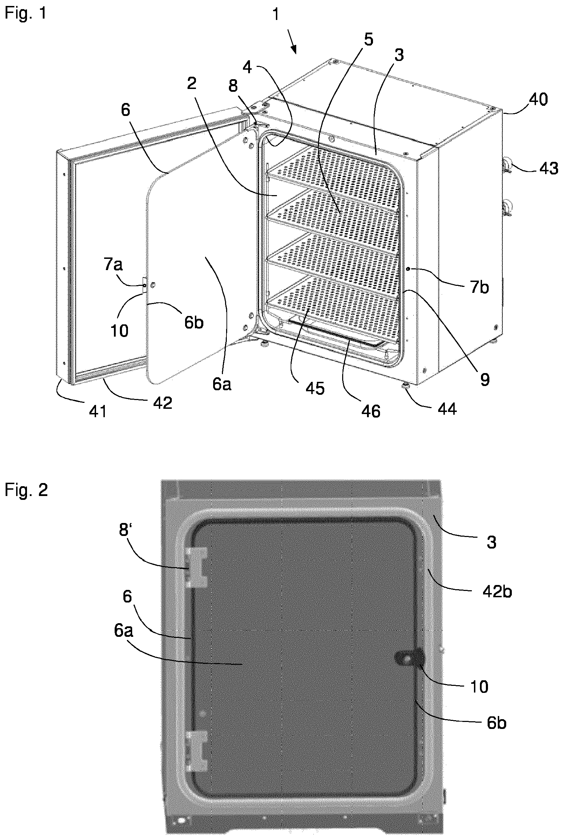

Fig. 1a zeigt eine als Temperierschrank bzw. Wärmeschrank ausgeführte Laborschrankvorrichtung 1 zum Lagern von Laborproben, genauer gesagt einen CO₂-Inkubator zur Lagerung von lebenden Zellkulturen in einer definierten Atmosphäre bei einer geregelten Temperatur, z.B. 37° C. Zu diesem Zweck ist der Kammerinnenraum 5 des Inkubators thermisch isoliert und gasdicht gegenüber der Umgebung abschließbar, die Gaszusammensetzung im Innenraum ist ebenfalls geregelt und kann über Gasanschlüsse 43 geändert werden. Das Kammergehäuse 2 des Inkubators steht auf Sockeln 44, kapselt den Innenraum 5 ein und mündet in der Frontseite 3 des Inkubators. Die Frontseite weist die Kammeröffnung 4 auf, durch die der Kammerinnenraum 5 zugänglich ist. Eine durchsichtige Kammertüre 6 dient dem Schließen der Kammeröffnung in einer Schließposition der Kammertüre. Beim Inkubator 1 ist das Kammergehäuse 2 innerhalb des Innenraums eines Außengehäuses 40 platziert, so dass das Kammergehäuse 2 und das Außengehäuse 40 voneinander beabstandet und gegeneinander thermisch isoliert sind. Im Kammerinnenraum sind Regalbleche 45 und eine Luftbefeuchterwanne 46 zu sehen. Die Frontseite 3 des Kammergehäuses und die Frontseite des Außengehäuses fallen vorliegend zusammen.

Die Außentüre 41 und die Kammertüre 6 sind in einer geöffneten Position gezeigt. Die Außentüre 41 ist über Scharniere an der Außenkante des Außengehäuses schwenkbar gelagert und weist eine umlaufende Silikondichtung 42 auf, die in der Schließposition der Außentüre und der Kammertüre an der Fronseite anliegt und so die Kammertüre umrahmt. Die Dichtung 42 kann alternativ oder zusätzlich auch als Dichtung 42b an der Frontseite 3 angebracht sein, siehe Fig. 2. Wie in Fig. 2 gezeigt ist, kann eine zusätzliche umlaufende Dichtung 42b an der Frontseite 3 vorgesehen sein, an der die Dichtung 42 in Schließposition anliegt.

Die Kammeröffnung 4 wird von einer Silikondichtung 9 eingerahmt, die an der Frontseite 3 angebracht ist, die alternativ oder zusätzlich aber auch als Dichtung 9' an der Innenseite der Kammertüre 6 angebracht sein kann.

Wenn die Außentüre 41 geöffnet wurde, ist die Kammertüre 6 des Inkubators zunächst noch geschlossen. Dazu dient die Verschlusseinrichtung (10, 7a, 7b). Bei geschlossener Kammertüre 6 kann der Benutzer zunächst den Innenraum 5 durch die transparente Türwand sichten, bevor der die Tür öffnet und eine Laborprobe einsetzt oder entnimmt.

Um die gelagerten Laborproben zu schützen, ist es bei Laborschrankvorrichtungen bzw. bei Temperierschränken bedeutsam, die Zeit zu minimieren, während der der Innenraum des gegenüber der Umgebung exponiert ist. Der vorliegenden Erfindung liegt die Beobachtung zugrunde, dass die Öffnungszeitintervalle durch die Gestaltung des Schließmechanismus beeinflussbar sind, mit dem die Kammertüre in der Schließposition gehalten und verschlossen wird. Es wurde deshalb eine Laborschrankvorrichtung mit einer Verschlusseinrichtung entwickelt, die vom Benutzer effizient zu bedienen ist, um die Öffnungszeit zu minimieren.

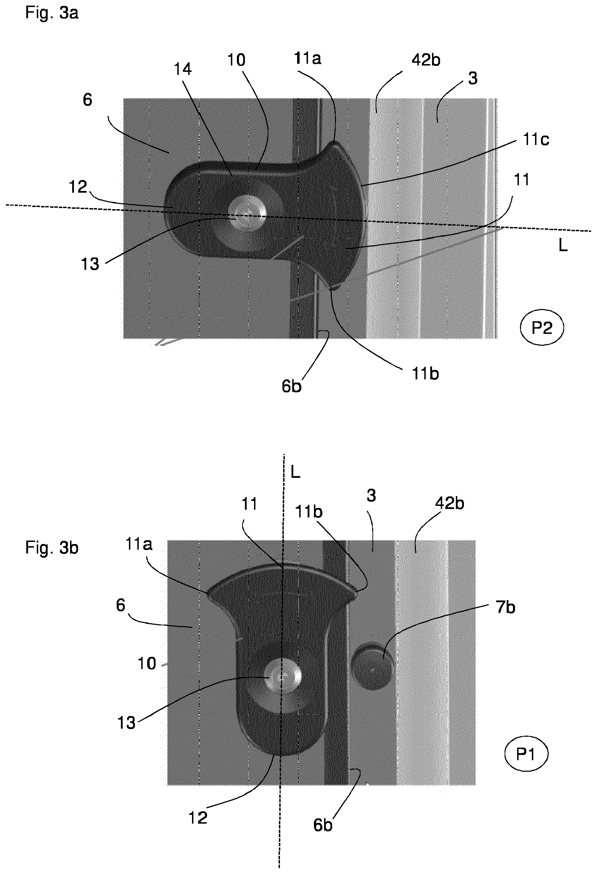

Die Verschlusseinrichtung dient dem Halten der Kammertüre 6 an der Frontseite 3 in der Schließposition durch eine magnetische Haltekraft und weist ein Betätigungselement 10, ein erstes Halteelement 7a und ein zweites Halteelement 7b auf. Das erste Halteelement 7a ist am Betätigungselement 10 befestigt und das zweite Halteelement 7b ist an der Frontseite 3 befestigt. Das Betätigungselement 10 ist schwenkbar an der Kammertüre 6 gelagert, so dass das Betätigungselement 10 zwischen einer ersten, geöffneten Position P1 (gezeigt in Fig. 3b), in der das erste und zweite Halteelement keine magnetische Haltekraft aufeinander ausüben, und einer zweiten, geschlossenen Position P2 (gezeigt in Fig. 3a), in der das erste und zweite Halteelement die magnetische Haltekraft aufeinander ausüben, schwenkbar beweglich ist. Das Betätigungselement 10 ist parallel zur Hauptebene 6a der Kammertüre 6 angeordnet und weist ein erstes Ende 11 auf. Dieses Ende des Betätigungselement 10 ist vorteilhaft als Grifflaschenabschnitt 11 ausgebildet, der in der zweiten Position über den Rand 6b der Kammertüre 6 hinausragt, so dass in der zweiten Position P2 die magnetische Haltekraft der Verschlusseinrichtung vom Benutzer sowohl durch Rotieren des Betätigungselements 10 von der zweiten Position P2 in die erste Position P1 überwindbar ist als auch durch Ziehen der Kammertüre mittels des Grifflaschenabschnitts in eine Richtung (A) senkrecht zur Hauptebene 6a der Kammertüre 6.

Durch die Ausgestaltung des Betätigungselements mit einem Grifflaschenabschnitt und der Möglichkeit, die magnetische Haltekraft sowohl durch Schwenken des Betätigungselements als auch durch Ziehen am Betätigungselemtent zu überwinden, wird eine kompakte und effizient bedienbare Verschlusseinrichtung realisiert. Durch die Verwendung der magnetisch miteinander wirkenden Halteelemente 7a, 7b entfällt die Anforderung herkömmlicher, durch mechanischen Formschluss wirkender Riegelverschlüsse, das Betätigungselement vor dem Öffnen der Kammertüre erst schwenken zu müssen bzw. das Betätigungselement vor dem Schließen der Kammertüre in einer geschwenkten Position halten zu müssen. Der Grifflaschenabschnitt erlaubt eine intuitive Bedienung des Betätigungselements. Nahezu jede vom Benutzer intuitiv getätigte Bewegung des Grifflaschenabschnitts führt zum gewünschten Öffnen bzw. Schließen des magnetischen Verschlusses. Während der Betriebszeit des Inkubators wird somit die Zeit reduziert, die die unterschiedlichen Benutzer benötigen, um die Bedienung des Kammertürverschlusses zu erlernen und zu vollziehen. Insbesondere ist eine einhändige Bedienbarkeit der Verschlusseinrichtung erleichtert, da die Bedienbarkeit unabhängig ist von den subjektiven Präferenzen des Benutzers, der Bedienung mit der rechten oder linken Hand oder auch mit einem anderen Körperteil oder Hilfsmittel, und unabhängig davon ob die Verschlusseinrichtung an der linken Seite oder rechten Seite der Kammertüre montiert ist.

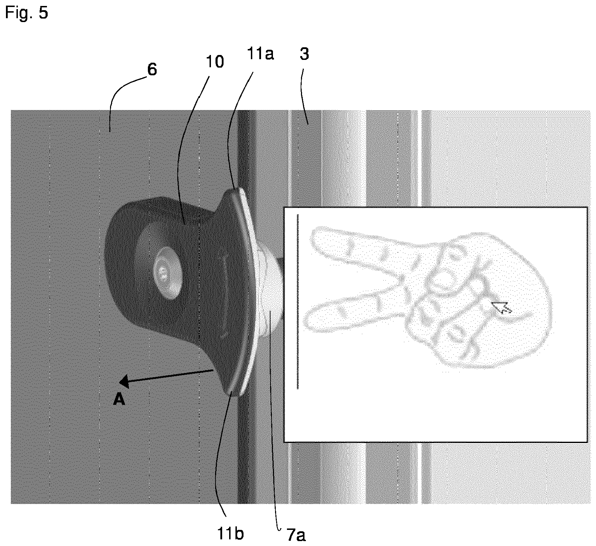

Das Betätigungselement 10 ist ein plattenförmiges Bauteil. Es weist eine Aussparung bzw. einen Hohlraum 16 auf, der hier vorteilhafter Weise zur Aufnahme weiterer funktioneller Bestandteile genutzt wird. Das Betätigungselement 10 weist einen langgestreckten Plattenkörper 14 auf, der als erstes Ende den Grifflaschenabschnitt 11 und als zweites Ende einen hier kreisförmig abgerundet geformten Endabschnitt 12 aufweist. Eine senkrecht zur Plattenebene des Betätigungselements gerichtetes Loch 15 dient der Aufnahme eines Drehachsenelements 13, mittels dem das Betätigungselement 11 schwenkbar an der Kammertüre 6 gelagert ist. Der Grifflaschenabschnitt 11 weist an seiner in Schließposition zur Frontseite 3 weisenden Unterseite (in Fig. 3a, 3b nicht sichtbar) das erste Halteelement 7a auf. Der Grifflaschenabschnitt 11 weist einen ersten Flügelabschnitt 11a und einen zweiten Flügelabschnitt 11 b auf, die integral mit dem Grifflaschenabschnitt 11 bzw. dem Betätigungselement 10 geformt sind. Die Flügelabschnitte 11 a und 11b springen gegenüber dem Plattenkörper 14 parallel zur Ebene des Plattenkörpers 14 bzw. zur Hauptebene 6a der Kammertüre seitlich ab, so dass ein Finger des Benutzers in Position P2 jeweils hinter dem ersten und/oder zweiten Flügelabschnitt eingreifen kann, um die Kammertüre durch Ziehen zu Öffnen. Der Grifflaschenabschnitt und die Flügelabschnitte springen insbesondere gegenüber dem ersten Halteelement 7a parallel zur Ebene des Plattenkörpers 14 bzw. zur Hauptebene 6a der Kammertüre seitlich ab, so dass das Hintergreifen des Grifflaschenabschnitts erleichtert wird. Dies ist in Fig. 5 gezeigt.

Das erste Halteelement 7a ist hier als ein Plattenelement aus magnetischem Edelstahl ausgeführt, dass in eine Aussparung auf der Unterseite des Grifflaschenabschnitts eingesetzt und eingeschraubt und/oder eingeklebt ist. Das Halteelement 7a kann auch gefedert gelagert am Betätigungselement 10 angeordnet sein, um eine Ausweichbewegung des Halteelements in Richtung A senkrecht zur Ebene des Plattenkörpers 14 bzw. zur Hauptebene 6a der Kammertüre zu ermöglichen. Alternativ könnte das erste Halteelement auch als Permanentmagnet ausgbildet sein oder mindestens einen solchen aufweisen. Die Polrichtung des Permanentmagneten kann in diesem Fall der Polrichtung des Permanentmagneten des zweiten Halteelements entsprechen, falls das zweite Halteelement auch als Permanentmagnet ausgbildet ist oder einen solchen aufweist, um die gewünschte anziehende Magnetkraft in Schließposition P2 zu erzeugen.

Das zweite Halteelement ist hier als Permanentmagnet ausgebildet, der in einer Fassung eingefasst und so geschützt ist, die an der Frontseite 3 befestigt ist. Der Permanentmagnet ist so gewählt, dass er die Betriebstemperatur des Temperierschranks toleriert. Im Falle des Inkubators, dessen Innenraum insbesondere bei Heiztemperaturen über 110° C sterilisiert wird, wurde deshalb als Material eine Samarium-Cobalt-Legierung gewählt. Das zweite Halteelement kann auch als ferromagnetischer Partner eines den Permanentmagneten aufweisenden ersten Halteelements ausgebildet sein.

In der Schließposition der Kammertüre und in der Position P2 des Betätigungselements liegen das erste und zweite Halteelement aneinander an und haften aufgrund der magnetischen Anziehungskraft aneinander. Durch Drehen des Betätigungselements aus der Position P2 in die Position P1 lässt sich diese Anziehungskraft deutlich leichter und komfortabler überwinden als durch Ziehen in Richtung A. In der geöffneten Position der Kammertüre 6, wenn das Betätigungselement 10 in der Position P2 angeordnet ist, führt das Schließen der Kammertüre automatisch zum Schließen des magnetischen Verschlusses, indem im Einflussbereich der Magnetkraft die magnetische Haftkraft das erste und zweite Halteelement direkt aufeinander zu bewegt. Das Anschlagen der Halteelemente ist zudem als "Klick" wahrnehmbar, so dass der Benutzer ein akustisches Feedback über das erfolgreiche Schließen der Kammertüre erhält.

Die Verschlusseinrichtung weist hier zudem eine Positionsrasteinrichtung 21, 25 auf, mittels der das Betätigungselement in der zweiten Position einrastet und in einer ersten Position einrastet. In der zweiten Position ist das Betätigungselement horizontal angeordnet, das heißt seine Längsachse L, siehe Fig. 3a, erstreckt sich horizontal. In der ersten Position ist das Betätigungselement vertikal angeordnet, das heißt seine Längsachse L, siehe Fig. 3b, erstreckt sich vertikal. In einer Aufsicht entlang der Achse A ist das Betätigungselement spiegelsymmetrisch zur Längsachse L ausgebildet. Die Positionsrasteinrichtung weist eine Rastelementenplatte 20 auf, auf der mindestens ein Rastelement 21 angeordnet ist. Im vorliegenden Fall sind drei äqidistant an der Rastelementenscheibe 20 entlang einer Kreisbahn B angeordnete Rastelemente 21, 21', 21" vorgesehen, wobei die Kreisbahn konzentrisch zur Lagerachse 23 der Rastelementenscheibe 20 angeordnet ist und mit der Kreisbahn des Höhenprofils übereinstimmt. Wenn das Betätigungselement bestimmungsgemäß an der Kammertüre montiert ist, wird die Rastelementenplatte 20 durch das Drehachsenelement an der Kammertüre fixiert, während das Betätigungselement 10 um die konzentrisch mit der Drehachse A angeordnete Gleithülse rotierbar angeordnet ist. Das Drehachsenelement 13 weist am in Richtung der Achse A gelegenen Ende einen Kopfabschnitt auf. Eine Mutter 19 ist vorgesehen, um die Rastelementenplatte 20 gegen die Kammertüre festzuklemmen, während das Betätigungselement rotierbar bleibt.

Das Rastelement 21 weist eine in Richtung der Achse A nach oben weisende Kugelhalterung 21b mit einer gefedert gelagerten Kugel 21a auf. Diese ist dazu angepasst, in Rastvertiefungen 25a einzugreifen, die in einem Höhenprofil 25 ausgebildet ist. Das Höhenprofil 25 ist vorliegend in den Hohlraum 16 bzw. in der Unterseite des Betätigungselements 10 integriert. Das Höhenprofil ist entlang einer Kreisringbahn 25 vorgesehen, die konzentrisch mit dem Loch 15 des Betätigungselements angeordnet ist. Eine erste Rastvertiefung 25a' kann vorgesehen sein, damit das Betätigungselement in der ersten Schwenkposition einrastet, die zweite Rastvertiefung 25a kann dazu vorgesehen sein, damit das Betätigungselement in der zweiten Schwenkposition einrastet. Das Höhenprofil ist zudem so ausgebildet, dass die Kugelhalterung des Rastelements an erhöhten Positionen 26a', 26a des Höhenprofils anschlägt, so dass das Betätigungselement, wenn dieses bestimmungsgemäß an der Kammertüre montiert ist, nur zwischen der ersten und zweiten Schwenkposition, und nicht darüber hinaus, schwenkbar ist. Die erste und zweite Schwenkposition stimmen jeweils mit der ersten und zweiten Rastposition überein. Um die gewünschte Stärke der Rastverbindung in der ersten und zweiten Schwenkposition zu erreichen, weist das Höhenprofil 25 entlang der Kreisbahn vorliegend drei im gleichen Abstand zueinander liegende Paare 25a', 25a von Rastvertiefungen auf, die jeweils von einer erhöhten Struktur 26a' getrennt sind. Grundsätzlich muss aber nur mindestens ein Paar 25a', 25a von Rastvertiefungen vorgesehen sein, um das Einrasten in der ersten und zweiten Schwenkposition zu erreichen.

Ein besonderes Merkmal des Betätigungselements ist vorliegend, dass die Positionsrasteinrichtung 21, 25, insbesondere das Höhenprofil 25, dazu eingerichtet ist, wahlweise so montiert zu werden, dass ein am rechten Rand der Kammertüre befestigtes Betätigungselement aus der zweiten Schwenkposition nur entgegen dem Urzeigersinn bis zur ersten Schwenkposition schwenkbar ist, oder dass ein am linken Rand der Kammertüre befestigtes Betätigungselement aus der zweiten Schwenkposition nur im Uhrzeigersinn bis zur ersten Schwenkposition schwenkbar ist. Dies wird durch die erhöhten Positionen 26a', 26a im Höhenprofil 25 erreicht, die vom fest an der Türe montierten Rastelement nicht passierbar sind. Grundsätzlich wäre auch eine andere Öffnungslogik möglich: es ist auch möglich, das Betätigungselement so zu montieren, dass es bei rechtsseitiger Montage aus der horizontalen (Position geschlossen) im Uhrzeigersinn nach unten in die vertikale (Position geöffnet) um 90° Grad geschwenkt wird. Bei linksseitiger Montage ist es auch möglich, das Betätigungselement so zu montieren, dass es aus der horizontalen (Position geschlossen) gegen den Uhrzeigersinn nach unten in die vertikale (Position geöffnet) um 90° Grad geschwenkt wird.

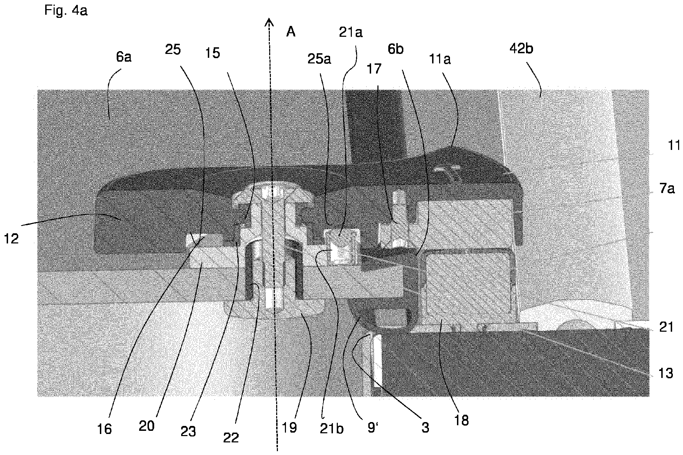

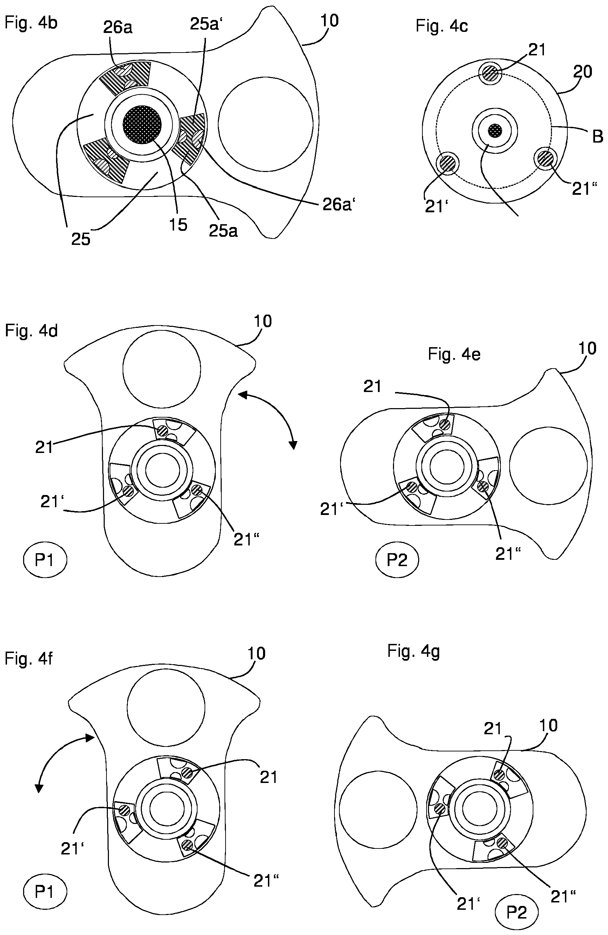

Ein weiteres besonderes Merkmal ist, dass das Betätigungselement 10 und insbesondere dessen Höhenprofil sowie die dazu komplementäre Rastelementenplatte mit Rastelementen so eingerichtet sind, dass das Betätigungselement und seine Rastelementenplatte wahlweise am linken oder rechten Rand einer Türe montiert werden kann. Diese Eigenschaft ergibt sich aus der Struktur und relativen Anordnung von Rastelementenplatte und Höhenprofil. In Fig. 4d ist ein Betätigungselement 10, das am rechten Rand einer Türe montiert ist, in der ersten Schwenkposition P1 gezeigt, in Fig. 4e ist es in der zweiten Schwenkposition P2 gezeigt. Die Position der Rastelementenplatte bzw. der Rastelemente 21, 21', 21", die fest an der nicht gezeigten Tür gelagert sind, sind in Fig. 4d und 4e identisch. In Fig. 4f ist dasselbe Betätigungselement 10 nun am linken Rand einer Türe montiert und in der ersten Schwenkposition P1 gezeigt, in Fig. 4e ist es in der zweiten Schwenkposition P2 gezeigt. Die Position der Rastelementenplatte bzw. der Rastelemente 21, 21', 21", die fest an der nicht gezeigten Tür gelagert sind, sind in Fig. 4f und 4g identisch. Der einzige Unterschied bei der Montage am rechten oder linken Rand einer Türe ist, dass in beiden Fällen zwar dieselbe Rastelementenplatte (bzw. dieselben Rastelemente) verwendet, diese aber bei Montage links an der Tür in einer gegenüber der rechtsseitigen Montage leicht verdrehten Position angeordnet ist. Dies ergibt sich unmittelbar aus den Figuren 4d bis 4g. Aufgrund dieser Eigenschaft ist das Betätigungselement 10 bzw. die so gestaltete erfindungsgemäße Laborschrankeinrichtung effizient und flexibel konstruierbar, da ein und dasselbe Betätigungselement 10 in Situationen verwendbar ist, in denen entweder ein linksseitiges Türscharnier oder ein rechtsseitiges Türscharnier benötigt wird.

## Patentansprüche

1. Laborschrankvorrichtung (1) zum Lagern von Laborproben, aufweisend
ein Kammergehäuse (2) mit einer Frontseite (3) und einer Kammeröffnung (4) der Frontseite, durch die der Kammerinnenraum (5) zugänglich ist, eine Kammertüre (6) zum Schließen der Kammeröffnung in einer Schließposition der Kammertüre,
eine Verschlusseinrichtung (10, 7a, 7b) zum Halten der Kammertüre an der Frontseite in der Schließposition durch eine magnetische Haltekraft, wobei die Verschlusseinrichtung ein Betätigungselement (10), ein erstes Halteelement (7a) und ein zweites Halteelement (7b) aufweist,
wobei das erste Halteelement (7a) am Betätigungselement (10) befestigt ist und das zweite Halteelement (7b) an der Frontseite (3) befestigt ist, und das Betätigungselement (10) schwenkbar an der Kammertüre (6) gelagert ist, so dass das Betätigungselement (10) zwischen einer ersten, geöffneten Schwenkposition (P1), in der das erste und zweite Halteelement keine magnetische Haltekraft aufeinander ausüben können, und einer zweiten, geschlossenen Schwenkposition (P2), in der das erste und zweite Halteelement die magnetische Haltekraft aufeinander ausüben können, schwenkbar beweglich ist,
wobei sich das Betätigungselement (10) parallel zur Hauptebene (6a) der Kammertüre (6) erstreckt und ein Ende aufweist, das als Grifflaschenabschnitt (11) ausgebildet ist, der in der zweiten Schwenkposition über den Rand (6b) der Kammertüre (6) hinausragt, so dass in der Schließposition der Kammertüre und in der zweiten Schwenkposition (P2) des Betätigungselements (10) die magnetische Haltekraft der Verschlusseinrichtung (10, 7a, 7b) vom Benutzer sowohl durch Rotieren des Betätigungselements (10) von der zweiten Schwenkposition (P2) in die erste Schwenkposition (P1) überwindbar ist als auch durch Ziehen der Kammertüre mittels des Grifflaschenabschnitts (11) in eine Richtung (A) senkrecht zur Hauptebene (6a) der Kammertüre (6).

2. Laborschrankvorrichtung gemäß Anspruch 1, wobei das erste und/oder das zweite Halteelement einen Permanentmagneten aufweisen oder aus einem solchen bestehen.

3. Laborschrankvorrichtung gemäß Anspruch 1 oder 2, wobei das Betätigungselement ein plattenförmiges Bauteil (10) ist, dessen Hauptebene parallel zur Schwenkebene und zur Hauptebene (6b) der Kammertüre (6) verläuft.

4. Laborschrankvorrichtung gemäß einem der vorangehenden Ansprüche, wobei das Betätigungselement ein Loch oder eine Aussparung aufweist, durch die ein senkrecht zur Schwenkachse des Betätigungselements ausgerichtetes Drehachsenelement angeordnet ist und das Betätigungselement schwenkbar mit der Kammertüre verbindet.

5. Laborschrankvorrichtung gemäß einem der vorangehenden Ansprüche, wobei das erste Halteelement im Bereich des Grifflaschenabschnitts (11) angeordnet ist, insbesondere an der in der ersten Schwenkposition (P1) der Kammertüre zugewandten Unterseite des Grifflaschenabschnitts.

6. Laborschrankvorrichtung gemäß einem der vorangehenden Ansprüche, wobei der Grifflaschenabschnitt mindestens einen Laschenabschnitt aufweist, der in der zweiten Schwenkposition (P2) in der Schwenkebene des Betätigungselements über die Kante (6b) der Kammertüre hinausragt.

7. Laborschrankvorrichtung gemäß Anspruch 6, wobei der mindestens eine Laschenabschnitt einen ersten Flügelabschnitt (11a) und einen zweiten Flügelabschnitt (11 b) beinhaltet, die senkrecht zur Längsrichtung (L) des Betätigungselements, welche der Radialrichtung der Schwenkbewegung entspricht, seitlich aus dem Betätigungselement hervorstehen.

8. Laborschrankvorrichtung gemäß einem der vorangehenden Ansprüche, wobei die Verschlusseinrichtung vorzugsweise eine Positionsrasteinrichtung (21, 25) aufweist, mittels der das Betätigungselement zumindest in der zweiten Schwenkposition einrastet und/oder zumindest auch in einer ersten Schwenkposition einrastet.

9. Laborschrankvorrichtung gemäß Anspruch 8, wobei die Positionsrasteinrichtung mindestens ein Rastelement (21a) und mindestens einen Rastabschnitt (25a) aufweist, die zumindest in der ersten und/oder der zweiten Schwenkposition miteinander verrasten.

10. Laborschrankvorrichtung gemäß einem der vorangehenden Ansprüche, der ein Temperierschrank zum Temperieren von Laborproben, insbesondere ein Inkubator für Zellkulturen ist.
